# EUROPÄISCHE PATENTSCHRIFT

(11) **EP 3 134 073 B1**
(45) Veröffentlichungstag und Bekanntmachung des Hinweises auf die Patenterteilung: **16.10.2024**
(21) Anmeldenummer: 15775073.8
(22) Anmeldetag: 22.04.2015
(51) Int. Cl.: A61K 9/00, A61K 47/10, A61K 31/4422, A61K 31/573, A61K 9/14, C08G 63/91, A61K 47/34

(54) **INJIZIERBARE UND IMPLANTIERBARE TRÄGERSYSTEME AUF BASIS VON MODIFIZIERTEN POLY(DIKARBONSÄURE-POLYOL ESTERN) ZUR KONTROLLIERTEN WIRKSTOFFFREISETZUNG**
INJECTABLE AND IMPLANTABLE CARRIER SYSTEMS BASED ON MODIFIED POLY(DICARBOXYLIC ACID POLYOL ESTERS) FOR THE CONTROLLED RELEASE OF ACTIVE INGREDIENT
SYSTÈMES SUPPORTS INJECTABLES ET IMPLANTABLES, À BASE DE POLYESTERS MODIFIÉS D'ACIDES DICARBOXYLIQUES AVEC DES POLYOLS, SERVANT À LA LIBÉRATION CONTRÔLÉE DE PRINCIPES ACTIFS

(30) Priorität: 23.04.2014 DE 102014005782
(43) Veröffentlichungstag der Anmeldung: 01.03.2017
(73) Patentinhaber: Martin-Luther-Universität Halle-Wittenberg, 06108 Halle/Saale (DE)
(72) Erfinder: MÄDER, Karsten, 04105 Leipzig (DE); WEISS, Verena, 16548 Glienecke (DE); KRESSLER, Jörg, 06114 Halle (Saale) (DE); NAOLOU, Toufik, 10717 Berlin (DE)
(74) Vertreter: Hertin und Partner Rechts- und Patentanwälte PartG mbB
(86) Internationale Anmeldenummer: PCT/DE2015/000204
(87) Internationale Veröffentlichungsnummer: WO 2015/161841

(56) Entgegenhaltungen:
- EP-A1- 1 270 024
- EP-A1- 1 374 860
- EP-A1- 2 623 537
- WO-A1-2005/059003
- WO-A2-02/38646
- WO-A2-2009/129155
- WO-A2-2009/148583
- US-A1- 2006 280 774
- SABINE KEMPE ET AL: "In situ forming implants an attractive formulation principle for parenteral depot formulations", JOURNAL OF CONTROLLED RELEASE, ELSEVIER, AMSTERDAM, NL, vol. 161, no. 2, 10 April 2012 (2012-04-10), pages 668 - 679, XP028492692, ISSN: 0168-3659, [retrieved on 20120418], DOI: 10.1016/J.JCONREL.2012.04.016

## Beschreibung

Trägersysteme, welche Wirkstoffe im Körper von Tieren oder Menschen kontrolliert freisetzen, sind von großer Bedeutung. Viele hoch wirksame Wirkstoffe zeichnen sich durch eine geringe Bioverfügbarkeit nach oraler Gabe und eine kurze Halbwertszeit aus, welche durch eine schnelle Ausscheidung (Elimination) oder Umwandlung (Metabolismus) des Wirkstoffes bedingt sind. Eine therapeutische Nutzung solcher Wirkstoffe ist nur durch geeignete Wirkstoffträgersysteme möglich, welche parenteral injiziert werden und die Wirkstoffe zeitlich kontrolliert freisetzen. Die Zeitspanne der Wirkstofffreisetzung ist von der Indikation abhängig. So kann eine Freisetzung von wenigen Stunden, Tagen, Wochen oder mehreren Monaten angestrebt werden. Typische Applikationsarten sind die subkutane, intraperitoneale oder intramuskuläre Applikation. Weiterhin werden solche Freigabesysteme zum Beispiel auch zur lokalen Therapie im Hirn, Auge, Ohr, Knochenmark und anderen Organen eingesetzt. Je nach Indikation können unterschiedliche Freisetzungsprofile, z. B. kontinuierlich, pulsatil angestrebt werden. Dabei soll das Wirkstoffträgersystem eine reproduzierbare, kontrollierte Freisetzung des Wirkstoffes gewährleisten und sich gegenüber dem Körper inert verhalten.

Gegenwärtig werden klinisch sowohl bioabbaubare als auch nicht abbaubare Trägermaterialien verwendet. Nichtbioabbaubare Materialien haben den Nachteil, dass sie aus dem Körper wieder entfernt werden müssen. Beispiele sind die Präparate Implanon^{®} (Firma Organon) und Vantas^{®} (Orion Pharma), welche nach 3 Jahren bzw. 12 Monaten aus dem Körper entfernt werden müssen und somit einen chirurgischen Eingriff erfordern. Als abbaubare Materialien werden hauptsächlich Polymilchsäure und Kopolymere aus Milch- und Glykolsäure genutzt. Die Monomere dieser Polymere sind alpha-Hydroxysäuren. Ein Nachteil dieser Polymere ist die Komplexität des Polymerabbaus. Dieser beruht auf einer autokatalytisch verlaufenden Hydrolyse, welche zu schwer steuerbaren Freisetzungskinetiken und sehr sauren pH-Werten (pH Wert 2) führen kann /1, 2, 3/. Nachteilig ist dabei, dass durch das saure Mikromilieu Wirkstoffe bereits vor ihrer Freisetzung zersetzt oder inaktiviert werden können /4,5/. Die meisten Marktpräparate sind entweder Mikropartikel oder vorgeformte Implantate (Rote Liste 2014, Rote Liste^{®} Service GmbH).

Mikropartikel, auch Mikrokapseln oder Mikrospherulen genannt, sind feste, meist sphärische Partikel mit einer typischen Größe im unteren Mikrometerbereich. Beispiele für bioabbaubare Mikropartikel sind Enantone^{®} (Takeda), Sixantone^{®} (Takeda), Decapeptyl^{®} (Ferring), Sandostatin^{®} LAR^{®}- (Sandoz), Pamorelin^{®} LA 3,75mg (Ipsen Pharma), Uropeptyl^{®} Depot (Uropharm), Risperdal^{®} Consta^{®} (Janssen-Cilag). Die Herstellung von Mikropartikeln mit einer engen und reproduzierbaren Größenverteilung ist sehr anspruchsvoll und aufwendig. Hierbei werden hauptsächlich die Koazervation, Sprühtrocknung oder Emulsionsverfahren mit Lösungsmittelentfernung angewandt /6/. Weiterhin müssen die Mikropartikel vor der Applikation mit dem Dispersionsmedium vermischt werden, was aufgrund der schlechten Benetzbarkeit der Partikel zeitaufwendig und oft problematisch ist. Unvollständiges Entleeren der Spritze und ein Verstopfen der Kanüle stellen weitere Probleme bei der Applikation von Mikropartikeln dar. Auch die Rückstandsproblematik organischer, zumeist chlorierter Lösungsmittel aus dem Herstellungsprozess, sowie die aufwändige Herstellung unter aseptischen Bedingungen und eine schwierige Herstellung im großtechnischen Maßstab setzen dieser Arzneiform Grenzen.

Beispiele für vorgeformte feste Implantate sind die Marktprodukte Zoladex^{®} (AstraZeneca), Profact ^{®} Depot (Sanofi), Leuprone^{®} HEXAL^{®} (Hexal), Leupro-Sandoz^{®} (Sandoz), Ozurdex^{®} (Allergan Pharmaceuticals). Bei den vorgeformten festen Implantaten besteht oft das Problem, dass eine relativ große Kanüle verwendet werden muss, um das Implantat, welches zudem oft als Fremdkörper empfunden wird und Schmerzen verursachen kann, in den Körper einzubringen.

Neuere Entwicklungen beschreiben sogenannte "in situ Implantate". Hierbei wird eine niedrigviskose Flüssigkeit in den Körper gespritzt, welche sich im Körper durch einen Stimulus (Trigger) in ein Depot (zum Beispiel in ein Gel oder einen Festkörper) umwandelt. Als Stimulus kann zum Beispiel ein Lösungsmittelaustausch, eine Thermogelierung oder eine chemische Reaktion (Quervernetzung) dienen /7/. Eine Quervernetzungsreaktion ist in vivo schwer zu kontrollieren und es treten häufig Toxizitätsprobleme mit den quervernetzenden Substanzen auf. Als wichtigste Vertreter für thermogelierende Systeme sind poloxamerbasierende Systeme, chitosanhaltige Zubereitungen und niedermolekulare pegylierte Polylaktide bzw. pegylierte Poly(laktid-co-glykolide) zu nennen. Nachteile der genannten thermogelierenden Systeme sind eine potentielle Gelierung vor der Applikation, Stabilitätsprobleme der Hilfsstoffe, eine zu schnelle Freisetzung und mangelnde Reproduzierbarkeit der Freisetzung. Im Fall der Systeme, welche auf Lösungsmittelaustausch basieren, gibt es im Gegensatz zu den anderen in situ Systemen kommerzielle Produkte. Ein typisches Beispiel ist Eligard^{®}, welches in Deutschland von der Firma Astellas vertrieben wird. Als Lösungsmittel wird N-Methylpyrrollidon (NMP) verwendet, in welchem das matrixbildende Polymer gelöst ist. Im Körper führt die gegenseitige Mischung von körpereigenem Wasser und NMP zur verminderten Löslichkeit und zum Präzipitieren des Polymers. Nachteile dieses Systems sind die den Polylaktiden und Poly(laktid-co-glycoliden) inherenten Probleme des autokatalytischen Polymerabbaus, welches oft schwer steuerbare und schwankende Freisetzungsraten bedingen.

Die Verwendung injizierbarer Polymere auf Basis von Hydroxyestern wird in WO 2007/012979 beschrieben. Nachteilig ist jedoch, dass eine kovalente Bindung des Wirkstoffes nur sehr eingeschränkt oder nicht ausreichend möglich ist, da nur die Endgruppen des Polymers modifiziert werden können. Weiterhin sind die im Patent WO 2007/012979 beschriebenen Polymere hinsichtlich ihrer Lipophilie nur sehr eingeschränkt steuerbar. Der Einsatz von bioabbaubaren Trägermaterialien, deren Lipophilie auf den Wirkstoff abgestimmt werden kann, wäre daher eine anzustrebende Zielsetzung.

Als Alternative zu polymerbasierten Systemen sind in der Literatur ebenfalls lipidbasierte Ansätze als parenterale Depotformen beschrieben. Hierbei werden durch Verpressen oder Extrusion von festen Lipiden wirkstoffhaltige Formkörper hergestellt. Nachteile von Lipidsystemen auf Basis von Glycerolfettsäureestern (Mono-, Di oder Triglyceride) oder Wachsen sind die eingeschränkte Steuerung der Lipophilie sowie das Auftreten von mehreren polymorphen Formen. Das komplexe polymorphe Verhalten beeinflusst sowohl die Reproduzierbarkeit der Wirkstoffbeladung und Wirkstofffreisetzung sowie die Abbaubarkeit der Wirkstoffträger. Das Patent DE 000003780862 **beschreibt feste Lipidkomprimate, die durch Implantation verabreicht werden. Das Patent** WO 2009/080275 **beschreibt feste Lipidimplantate, welche durch Extrusion hergestellt werden.**

**Weiterhin wird die Herstellung von Lipidkomprimaten und Lipidextrudaten im Patent** WO 2005/102284 **beschrieben.** Die EP 2 623 537 A1 offenbart Polykondensate erhältlich durch Veresterung von Dikarbonsäuren mit Diolen. Die WO2009/148583 offenbart bioabbaubare thermoplastische Polymere auf Basis von Polyestern aus Diolen mit Dikarbonsäuren. Die EP1 374 860 A1 offenbart Polymerwachse, deren Rückgrat Polyester aus Dikarbonsäuren mit Polyolen besteht. Die EP 1 270 024 A1 offenbart medizinische Hilfsmittel, die aus Polymerwachs bestehen, das aus Dikarbonsäuren und Polyolen durch Veresterung erhältlich ist.

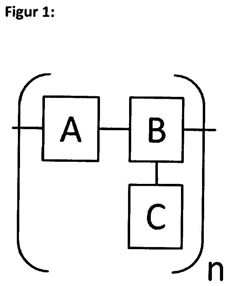

**Aufgabe der Erfindung war es, eine Alternative zu den bisher klinisch genutzten bioabbaubaren Polymeren auf Basis von Monomeren mit Hydroxykarbonsäurestruktur aufzuzeigen. Das Problem wurde erfindungsgemäß durch lineare Polyester gelöst, welche durch Veresterung von Dikarbonsäuren und mehrwertigen Alkoholen (Diole, Triole oder höherwertige Alkohole) entstehen (****Figur 1****)**.

Werden als Alkoholkomponente Substanzen mit drei oder einer größeren Anzahl von Hydroxylgruppen genutzt, so sind bei geeigneter Synthesestrategie nach der Polymerisation im Polymerrückgrat freie Hydroxylgruppen verfügbar. Hierbei werden enzymatische Umsetzungen unter Verwendung divinylaktvierter Dikarbonsäuren bevorzugt, da durch diese definierte lineare Polymere mit definierten freien Hydroxylgruppen erhalten werden und eine Quervernetzung vermieden wird /8/.

Die freien Hydroxylgruppen können entweder mit Wirkstoffen oder mit weiteren Hilfsstoffen kovalent chemisch umgesetzt werden

Zur kovalenten Verknüpfung mit Wirkstoffen ist bisher wenig bekannt. Shafioul et al. publizierten die chemische Verknüpfung des Wirkstoffes Xanthorrhizol an Poly(dikarbonsäure-multiol ester) und seine enzymatische Freisetzung /9/. Hingegen wird eine Veresterung von Poly(dikarbonsäure-multiol estern) mit Fettsäuren mehrfach beschrieben /10-16/, wobei die Verwendung von fettsäuremodifzierten Poly(dikarbonsäure-multiol estern) zur Herstellung von wirkstoffhaltigen Mikro- und Nanopartikeln *in vitro* dargestellt wird. Die Herstellung von Mikro- und Nanopartikeln ist jedoch aufwendig und die Partikelgröße oft schwer kontrollierbar.

Erfindungsgemäß wurde erstens gefunden, dass modifizierte Poly(dikarbonsäure-multiol ester) ohne weitere Zusätze als injizierbare Implantate geeignet sind. Hierbei können die Polymere ohne **Verwendung eines organischen Lösungsmittels direkt injiziert werden.** Die Erfindung betrifft injizierbare Trägersysteme auf Basis modifizierter aliphatischer Polyester zur Anwendung am Menschen oder am Tier mit dem Ziel der kontrollierten Freisetzung einer bioaktiven Substanz zu therapeutischen und / oder diagnostischen Zwecken bestehend aus Polymeren, deren Grundstruktur durch einen kammartigen Aufbau aus den Strukturelementen (A), (B) und (C) gekennzeichnet ist, wobei den Polymerrückgrad die Strukturelemente (A) und (B) bilden, welche aus aliphatischen Dikarbonsäuren (A) und aus Polyolen (B), die drei oder mehr OH-Gruppen enthalten, bestehen, wobei zwei Hydroxylgruppen des Polyols (B) mit den Dikarbonsäuren des Strukturelements (A) zu einem linearen Polymerrückgrat verestert sind, nach Ausbildung des aus den Strukturelementen (A) und (B) bestehenden und durch Esterbindungen verknüpften Polymerrückgrates bei Verwendung von Triolen und höherwertigen Polyolen im Strukturelement (B) noch eine (bei Triolen) oder mehrere (Anzahl der Hydroxylgruppen im Polyol > 3) freie Hydroxylgruppen vorhanden sind, die vollständig oder teilweise mit aliphatischen Fettsäuren und / oder bioaktiven Substanzen kovalent oder nicht kovalent verknüpft sind (Strukturelement (C)).

**Des Weiteren wurde zweitens gefunden, dass modifizierte Poly(dikarbonsäure-multiol ester) mit geeigneten biokompatiblen organischen Lösungsmitteln versetzt und injiziert werden können.**

### Ausführungsbeispiele:

### Beispiel 1:

Fettsäuremodifizierte Poly(glycerol-adipate) können durch folgendes oder ähnliche Verfahren synthetisiert werden /10-16/.

Als Strukturelement (A) (Dikarbonsäure) wird Adipinsäure verwendet, als Polyol (Strukturelement (B)) dient Glycerol, welches drei Hydroxylgruppen besitzt (Figur 1). Das Polymerrückgrad Polyglyceroladipat wird zum Beispiel durch eine enzymatische Reaktion von Divinyladipat oder

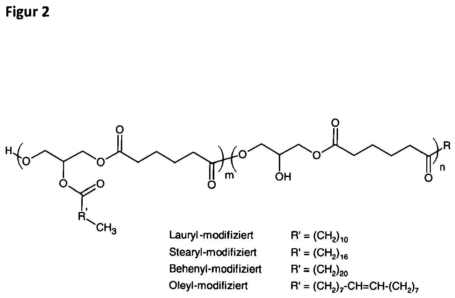

Dimethyladipat mit Glycerol in Tetrahydrofuran oder einem anders geeigneten Lösungsmittel durchgeführt /10/. Beispielhaft verwendete Fettsäuren sind Laurin-, Stearin-, Behen- oder Ölsäure. Der Substitutionsgrad der Fettsäuren beträgt zwischen 1% und 100 % (bezogen auf die nach Hydroxylgruppen des Poly(glycerol-adipats) (Figur 2).

Die öl- und laurylsäuremodifizierten Polymere besitzen bei einer Temperatur von 20°C (Raumtemperatur) eine flüssige Konsistenz. Weiterhin werden nicht-erfindungsgemäss offenbart: stearin- und behensäuremodifizierten Polymere besitzen bei 20°C eine feste Konsistenz. Sie schmelzen im Bereich von 30-45 °C (stearylmodifiziertes Poly(glyceroladipat)) bzw. zwischen 50°C und 65°C (behenylmodifiziertes Poly(glyceroladipat)).

Bei den behenyl- und stearylmoldifizierten Poylmeren kann durch Schmelz- oder Extrusionsprozesse sehr einfach ein implantierbarer Formling gewonnen werden.

### Beispiel 2:

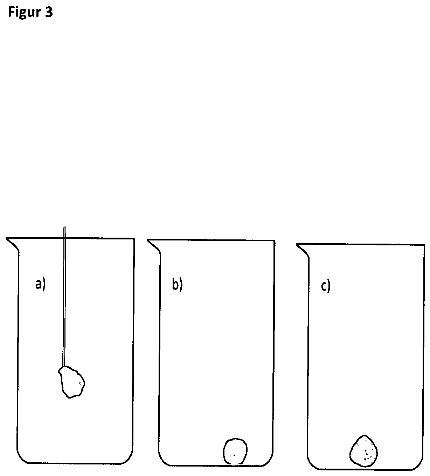

Laurylsäuremodifiziertes Poly(glyceroladipat) (Veresterungsgrad 25 % bezogen auf freie Hydroxylgruppen des Polyglycerinadipats) wird durch eine 25 Gauge Nadel in phosphatgepufferter Kochsalzlösung (PBS, 1:10 verdünnt) injiziert. Es bildet sich ein kugelförmiges Depot (Figur 3).

### Beispiel 3:

Laurylsäuremodifiziertes Poly(glyceroladipat) (Veresterungsgrad 95 % bezogen auf freie Hydroxylgruppen des Polyglycerinadipats) wird durch eine 25 Gauge Nadel in phosphatgepufferter Kochsalzlösung (PBS, 1:10 verdünnt) injiziert. Es bildet sich ein kugelförmiges Depot.

### Beispiel 4:

Ölsäuremodifiziertes Poly(glyceroladipat) (Veresterungsgrad 15 % bezogen auf freie Hydroxylgruppen des Polyglycerinadipats) wird durch eine 25 Gauge Nadel in phosphatgepufferter Kochsalzlösung (PBS, 1:10 verdünnt) injiziert. Es bildet sich ein kugelförmiges Depot.

### Beispiel 5:

Ölsäuremodifiziertes Poly(glyceroladipat) (Veresterungsgrad 92 % bezogen auf freie Hydroxylgruppen des Poly(glycerinadipats)) wird durch eine 25 Gauge Nadel in phosphatgepufferter Kochsalzlösung (PBS, 1:10 verdünnt) injiziert. Es bildet sich ein kugelförmiges Depot.

### Beispiel 6:

Fettsäuremodifizierte Poly(glycerol-sebacat) Polymere werden analog den in /10/ publizierten Synthesewegen hergestellt. Als Strukturelement (A) (Dikarbonsäure) wird Sebacinsäure verwendet, als Polyol (Strukturelement (B)) dient Glycerol, welches drei Hydroxylgruppen besitzt (Figur 1). Das Polymerrückgrad Poly(glycerol-sebacat) wird zum Beispiel durch eine enzymatische Reaktion von Divinylsebacat mit Glycerol in Tetrahydrofuran oder einem anders geeigneten Lösungsmittel durchgeführt /10/. Beispielhaft verwendete Fettsäuren sind Laurin-, Stearin-, Behen- oder Ölsäure. Der Substitutionsgrad der Fettsäuren beträgt zwischen 1% und 100 % (bezogen auf die nach Hydroxylgruppen des Poly(glycerol-sebacat)

### Beispiel 7

### Stearylmodifiziertes Poly(sorbitol-adipat)

Das Polymerrückgrad wird aus dem Polyol Sorbitol und der Dikarbonsäure Adipinsäure analog den für Poly-(Glycerol-adipat) publizierten Vorschriften hergestellt und mit Stearinsäure modifiziert. Bei einem Veresterungsgrad von 30 % (Veresterungsgrad 30 % bezogen auf freie Hydroxylgruppen des Poly(sorbitol-adipats) entsteht ein bei Raumtemperatur festes Polymer, welches bei 43°C schmilzt.

Durch Schmelz- oder Extrusionsprozesse kann sehr einfach ein implantierbarer Formling gewonnen werden.

### Beispiel 8:

In vitro Freisetzung des Fluoreszenzfarbstoffes 1,1'-Dioctadecyl-3,3,3',3'-tetramethylindocarbocyanine perchlorate (DII): Laurylsubstituierte Poly(glycerol-adipat) Polymere wurden mit dem Fluoreszenzfarbstoff DII beladen (1% m/m) und 60 Mikroliter bei 37°C in vitro in PBS inkubiert.

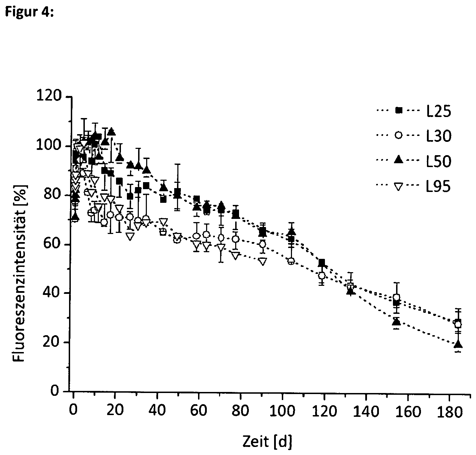

Die folgenden Kurven in Figur 4 zeigen eine kontrollierte Freisetzung des Modellstoffes in vitro. Die Zahl hinter dem L symbolisiert den Substitutionsgrad der Laurinsäure bezogen auf freie Hydroxylgruppen des Poly(glycerin-adipats).

### Beispiel 9:

### In vivo Freisetzung des Fluoreszenzfarbstoffes DII aus laurylsubstituierten Poly(glycerol-adipat)

Die Polymere wurden mit dem Fluoreszenzfarbstoff 1,1'-Dioctadecyl-3,3,3',3'-tetramethylindocarbocyanine perchlorate (DII) (0,5-1 % m/m) beladen und 50 Mikroliter subkutan in SKH1-Mäuse injiziert .

In vivo Fluoreszenzbilder von den DII beladenen laurylmodifizierten Poly(glycerol-adipat) Polymeren nach subkutaner Injektion (50 Mikroliter) in SKH1 Nacktmäusen belegten durch sichtbare Signale die in vivo Freisetzung. Der Substitutionsgrad der Laurinsäure betrug 95 % bzw. 25%.

Die Ergebnisse zeigen eine kontrollierte in vivo Freisetzung über mehrere Wochen bis Monate. Hierbei wurde überraschenderweise gefunden, dass das lipophilere Implantat (Substitutionsgrad 95 % bezogen auf freie Hydroxylgruppen) rascher freisetzt als das niedrig substituierte Implantat (25 % bezogen auf freie Hydroxylgruppen). Die Ergebnisse belegen, dass die Freisetzung von Stoffen in vivo durch den Substitutionsgrad mit der Fettsäure gesteuert werden kann.

### Beispiel 10:

Herstellung flüssiger Dexamethason-haltiger Implantate. Dexamethason wird in fettsäuremodifiziertes Poly(glycerol-adipat) in einer Konzentration von 10 % m/m eingearbeitet. Bei den flüssigen Polymeren (lauryl- und oleylmodifiziertes Poly(glycerol-adipat) kann die Einarbeitung bei Raumtemperatur erfolgen. Die dexamethasonbeladenen (lauryl- und oleylmodifiziertes Poly(glycerol-adipat) Polymere können aufgrund ihrer flüssigen Konsistenz injiziert werden.

### Referenzbeispiel

Herstellung fester Dexamethason-haltiger Implantate. Dexamethason wird in fettsäuremodifiziertes Poly(glycerol-adipat) in einer Konzenraton von 20 % m/m eingearbeitet. Die bei Raumtemperatur festen Polymere (z.B. stearylmodifiziertes und behenylmodifiziertes Poly(glycerol-adipat)) werden geschmolzen und der Wirkstoff wird homogen eingearbeitet. Durch Schmelzen und Erstarren, Verpressen oder Extrudieren kann bei den festen Polymeren ein geeigneter Formling (z.B. Blättchen oder Stäbchen) erhalten werden, welcher implantiert werden kann.

### Referenzbeispiel 12:

**Nifidipin wird in stearylmodifiziertes Poly(sorbitol-adipat) eingearbeitet in einer Konzentration von 10 % m/m eingearbeitet. Hierzu wird das Polymer bei 50 °C geschmolzen und der Wirkstoff unter Lichtschutz homogen verteilt. Das geschmolzene wirkstoffhaltige Polymer wird durch Erstarren in einer geeigneten Form in die gewünschte Größe und Form gebracht.**

### Referenzbeispiel 13:

**Wirkstoffhaltiges in situ Implantat. Stearylmodifiziertes Poly(glycerol-adipat) wird mit N-**Methylpyrrollidon (NMP) in einem Verhälntis von 1:1 (m/m) gemischt. Leuprolinacetat wird in einer Konzentration von 5 % (m/m) homogen eingearbeitet. Die Zubereitung kann durch eine 25 Gauge Kanüle verabreicht werden und bildet nach Kontakt mit Wasser oder im Körperinneren in situ ein Implantat aus.

### Referenzbeispiel 14:

Implantat mit kovalent gebundener Betulinsäure. Das Polymerrückgrat aus Poly(glycerol-adipat) wird nach den in der Literatur beschriebenen Vorschriften synthetisiert /10/. Anstelle der in der Literatur /10/ beschriebenen Fettsäuren wird in einer analogen Reaktion die bioaktive Substanz Betulinsäure kovalent an das Polymerrückgrat geknüpft. Das entstandene Betulinsäure-Poly(glycerol-adipat) kann entweder durch Schmelzen in eine gewünschte Implantatform überführt werden oder mit Hilfe eines bioverträglichen organischen Lösungsmittels (z.B. NMP) als in situ Implantat appliziert werden.

### Literaturverzeichnis

/1/ Mäder et al., Biomaterials, 17(4), 457-461 (1996)
/2/ K Mäder, G Bacic, A Domb, O Elmalak, R Langer and HM Swartz. J Pharm Sei 86: 126, (1997).
/3/ Liu und Schwendeman, Molecular Pharmaceutics, 9(5), 1342-1350(2012)
/4/ A Lucke, J Kiermaier and A Göpferich. Pharm Res 19: 175, (2002).
/5/ A Lucke and A Göpferich. Eur J Pharm Biopharm 55: 27, (2003).
/6/ Beck-Broichsitter et al., Parenterale Depotarzneiformen-Mikropartikel, in: Innovative Arzneiformen, herausgegeben von Mäder und Weidenauer, Wissenschaftliche Verlagsgesellschaft Stuttgart, S. 235-262, (2010)
/7/ S Kempe and K Mäder. J Control Release 161: 668, (2012)
/8/ BJ Kline, EJ Beckman, AJ Russell. J Am Chem Soc 120: 9475 (1998)
/9/ Shafioul, Azam Sharif Mohammed; Pyo, Jung In; Kim, Kwan Soo; Cheong, Chan Seong: Synthesis of poly (glycerol-co-dioate-co-butanedioate-co-xanthorrhizol) ester and a study of chain length effect on pendant group loading. Journal of Molecular Catalysis B: Enzymatic (2012), 84, 198-204., DOI:10.1016/j.molcatb.2012.05.022
/10/ Naolou, T.; Weiss, V. M.; Conrad, D.; Busse, K.; Maeder, K.; Kressler, J.: Fatty acid modified poly(glycerol adipate) - polymeric analogues of glycerides. ACS Symposium Series(2013), 1135 (Tailored Polymer Architectures for Pharmaceutical and Biomedical Applications), 39-52, 14 pp., DOI:10.1021/bk-2013-1135.ch004
/11/ Kressler, Joerg; Naolou, Toufik; Conrad, Delf; Busse, Karsten; Amado, Elkin; Weiss, Verena; Maeder, Karsten: Fatty acid modified poly(glycerol adipate)-polymeric analogues of glycerides. Polymer Preprints (American Chemical Society, Division of Polymer Chemistry) (2012), 53(1), 567-568.
/12/ Weiss, Verena M.; Naolou, Toufik; Amado, Elkin; Busse, Karsten; Maeder, Karsten; Kressler, Joerg Formation of Structured Polygonal Nanoparticles by Phase-Separated Comb-Like Polymers. Macromolecular Rapid Communications(2012), 33(1), 35-40., DOI:10.1002/marc.201100565
/13/ Orafai, Hossein; Kallinteri, Paraskevi; Garnett, Martin; Huggins, Sean; Hutcheon, Gillian; Pourcain, Cristopher Novel poly(glycerol-adipate) polymers used for nanoparticle making: a study of surface free energy. Iranian Journal of Pharmaceutical Research (2008), 7(1), 11-19
/14/ Puri, Sanyogita; Kallinteri, Paraskevi; Higgins, Sean; Hutcheon, Gillian A.; Garnett, Martin C.: Drug incorporation and release of water soluble drugs from novel functionalized poly(glycerol adipate) nanoparticles. Journal of Controlled Release (2008), 125(1), 59-67
/15/ Kallinteri, Paraskevi; Higgins, Sean; Hutcheon, Gillian A.; St. Pourcain, Christopher B.; Garnett, Martin C.: Novel Functionalized Biodegradable Polymers for Nanoparticle Drug Delivery Systems. Biomacromolecules (2005), 6(4), 1885-1894
/16/ WO 2004096178 A1 20041111 - Garnett, Martin Charles; Hutcheon, Gillian; Higgins, Sean; Kallinteri, Paraskevi; St. Pourcain, Chris: Nano and microparticle drug delivery systems comprising polyesters containing aliphatic dicarboxylate residues and residues of aliphatic polyols

### Erläuterungen zu den Figuren

**Figur 1****:**
   Vereinfachte (links) und detailliertere (rechts) Grundstruktur von modifizierten Poly(dikarbonsäuremultiol estern).
   Strukturelement (A): Dikarbonsäure
   Strukturelement (B): Polyol (Diol, Triol oder höherwertiges Polyol)
   Strukturelement ^{©}: kovalent gebundener Wirkstoff oder Hilfsstoff
   Der bioabbaubare Polymerrückgrad entsteht aus der Veresterung von Dikarbonsäuren (Strukturelement (A)) und Polyolen (Strukturelement (B)). Hierbei werden zwei Hydroxylgruppen des Polyols verestert. Bei höherwertigen Polyolen (Anzahl der Hydroxylgruppen größer / gleich drei) können die nach Bildung des Polymerrückgrates noch freien Hydroxylgruppen kovalent mit bioaktiven Substanzen oder Hilfsstoffen (z. B. Fettsäuren) teilweise oder vollständig kovalent verbunden werden (Strukturelement (C)).
**Figur 2****:**
   Strukturbeispiele von fettsäuremodifizierten Poly(glycerol-adipat). Das Polymerrückgrat ist aus Poly(glyceroladipat) aufgebaut. Ein Teil der nach Bildung des Polymerrückgrates noch freien Hydroxylgruppen ist mit Fettsäuren (Laurinsäure, Stearinsäure, Behensäure, Ölsäure) verestert.
**Figur 3****:**
   Schematische Abbildung des Verhaltens von laurylsubstituierten Poly(gylceroladipat) Polymeren nach einer in vitro Injektion mit einer 25 Gauge Nadel in phosphatgepufferte Kochsalzlösung (PBS Puffer, pH 7,4 1:10 verdünnt mit bidestillierten Wasser). (a); nach einer Minute (b) und 6 Stunden (c) nach Injektion. Das Implantat sinkt nach Injektion zu Boden und bildet eine kugelähnliche Form aus.
**Figur 4****:**
   In vitro Zeitabhängigkeit der Fluoreszenzintensität von 1,1'-Dioctadecyl-3,3,3',3'-tetramethylindocarbocyanine perchlorate (DII) beladenen laurylsubstitutierten Poly(glyceroladipinsäure) Polymeren. Der Modellstoff wird in vitro kontrolliert über mehrere Wochen freigesetzt.

## Patentansprüche

1. Injizierbare Trägersysteme auf Basis modifizierter aliphatischer Polyester zur Anwendung am Menschen oder am Tier mit dem Ziel der kontrollierten Freisetzung einer bioaktiven Substanz zu therapeutischen und / oder diagnostischen Zwecken bestehend aus Polymeren, deren Grundstruktur durch einen kammartigen Aufbau aus den Strukturelementen (A), (B) und (C) gekennzeichnet ist,
wobei den Polymerrückgrad die Strukturelemente (A) und (B) bilden, welche aus aliphatischen Dikarbonsäuren (A) und aus Polyolen (B), die drei oder mehr OH-Gruppen enthalten, bestehen, wobei
zwei Hydroxylgruppen des Polyols (B) mit den Dikarbonsäuren des Strukturelements (A) zu einem linearen Polymerrückgrat verestert sind, nach Ausbildung des aus den Strukturelementen (A) und (B) bestehenden und durch Esterbindungen verknüpften Polymerrückgrates bei Verwendung von Triolen und höherwertigen Polyolen im Strukturelement (B) noch eine (bei Triolen) oder mehrere (Anzahl der Hydroxylgruppen im Polyol > 3) freie Hydroxylgruppen vorhanden sind, die vollständig oder teilweise mit aliphatischen Fettsäuren und / oder bioaktiven Substanzen kovalent oder nicht kovalent verknüpft sind (Strukturelement (C)).

2. Trägersysteme nach Anspruch 1 **gekennzeichnet dadurch, dass** die aliphatischen Dikarbonsäuren des Strukturelements (A) der folgenden allgemeinen Struktur 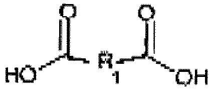 zwischen den beiden Karboxylgruppen einen Molekülteil R₁ aufweisen, der eine Alkylstruktur besitzt und gesättigt oder ungesättigt, verzweigt oder unverzweigt mit R₁= 0 (null) und ≤ 40 (vierzig) Kohlenstoffatome ist, vorzugsweise 1 (eins) bis 14 (vierzehn) Kohlenstoffatome, besonders bevorzugt 4 (vier) bis 10 (zehn) Kohlenstoffatome.

3. Trägersysteme nach Anspruch 1 **gekennzeichnet dadurch, dass** das Polyolstrukturelement (B) aus einem Gemisch von Triolen und höherwertigen Polyolen bzw. Mischungen derselben bestehen kann.

4. Trägersysteme nach Anspruch 1 und 3**gekennzeichnet dadurch, dass** als Polyole Glycerol, Erythritol, Xylitol, Mannitol, Sorbitol, Laktose, Sukrose (Saccharose), Trehalose, Glukose, Maltose, Isomalt und/oder Maltitol zur Anwendung kommen.

5. Trägersysteme nach den Ansprüchen 1, 3 und 4 **gekennzeichnet dadurch, dass** das Polyol (B) mit weiteren Substanzen mittels des Strukturelementes (C) kovalent verknüpft ist, so dass eine kammartige Polymerstruktur entsteht, wobei der Verknüpfungsgrad zwischen 0,1 und 100 % der nach Bildung des Polymerrückgrates noch vorhandenen freien Hydroxylgruppen beträgt.

6. Trägersysteme nach den Ansprüchen 1 bis 5 **gekennzeichnet dadurch, dass** das Strukturelement (C) eine Karbonsäure oder eine oder mehrere bioaktive Substanz/en ist, welche durch eine Esterbindung mit der Polyolstruktur (B) verknüpft ist, wobei die Fettsäure gesättigt oder ungesättigt ist und der Veresterungsgrad zwischen 0 (null) und 99,9 % der freien Hydroxylgruppen beträgt.

7. Trägersysteme nach den Ansprüchen 1 bis 6 **gekennzeichnet dadurch, dass** die Anzahl der Kohlenstoffe der Karbonsäure zwischen 2 (zwei) und 22 (zweiundzwanzig) beträgt, insbesondere zwischen 8 (acht) und 22 (zweiundzwanzig) Kohlenstoffatomen, wie Caprylsäure (Oktansäure), Caprinsäure (Dekansäure), Laurinsäure (Dodekansäure), Myristinsäure (Tetradekansäure), Palmitinsäure (Hexadekansäure), Stearinsäure (Oktadekansäure), Ölsäure ((9Z)- Octadeca- 9- ensäure), Linolsäure ((9Z,12Z)-Octadeca- 9,12-diensäure), Linolensäure ((9Z,12Z,15Z- Octadeca- 9,12,15- triensäure).

8. Trägersysteme gemäß der Ansprüche 1 bis 7, in denen die bioaktive Substanz/en eingearbeitet (nicht kovalent gebunden) oder kovalent gebunden enthalten ist, wobei im Fall einer Einarbeitung (nichtkovalente Bindung) die bioaktive Substanz/en gelöst, emulgiert oder suspendiert vorliegen und im Fall einer kovalenten Bindung die bioaktive Substanz/en durch eine chemische Reaktion einer in geeigneten funktionellen Gruppen der bioaktiven Substanz/en, z.B. einer Säuregruppierung, mit der im Strukturelement (B) vorhandenen freien Hydroxylgruppen kovalent durch eine Esterbindung an das polymere Trägermaterial gebunden ist.

9. Trägersysteme nach Anspruch 8 **gekennzeichnet dadurch, dass** die enthaltenen bioaktiven Substanzen mit einem niederen, mittleren oder hohen Molekulargewicht hydrophiler, amphiphiler oder hydrophober Natur sind und gelöst, emulgiert oder suspendiert vorliegen.

10. Trägersysteme nach Anspruch 8 und 9 **gekennzeichnet dadurch, dass** als bioaktive Substanzen Stoffe aus der Gruppe der Antibiotika, Antiinfektiva, Zytostatika oder andere antineoplastische Mittel und Protektiva, Lokalanästhetika, Analgetika, Neuropharmaka, Hormone, Immunmodulatoren, Imrnunsuppressiva, Antimykotika, Antiinflarnmatoria, Analgetika, Antihämorrhagika, Antifibrinolytika, Vitamine, Hämostatika, Antiphlogistika, Betarezeptoren- und Calciumkanalblocker, Corticokie, Entwöhnungsmittel/Mittel zur Behandlung von Suchterkrankungen, Fibrinolytika, Antihypoxämika, Herz-Kreislauf-Mittel, Antikoagulantia, Antiparasitäre Mittel, Antikariesmittel, Antiparodontosemittel, Ophthalmika, Otologika, Sexualhormone und ihre Hemmstoffe, Parkinsonmittel und andere Mittel gegen extrapyramidale Störungen, Psychopharmaka, Sera, Immunglobuline, Impfstoffe, Mineralstoffe, Thrombozytenaggregationshemmer, Tuberkulosemittel, Diuretika, Magen-Darm-Mittel, Antiadiposita, Analeptika, Antirheumatika, Antiallergika, Antianämika, Antiarrhythmika, Antidementiva, Antidiabetika, Antidota, Antiemetika, Antivertiginosa, Antiepileptika, Antihypertonika, Antihypoglykämika, Antihypotonika, Antitussiva, Expektorantia, Arteriosklerosemittel, Hemmstoffe des Renin-Angiotensin-Aldosteron-Systems, Broncholytika, Antiasthmatika und andere Mittel für den Respirationstrakt, Cholinergika, Antiseptika, Diagnostika und Mittel zur Diagnosevorbereitung, Diuretika, Enzyminhibitoren, Präparate bei Enzymmangel und Transportproteine, Geriatrika, Gichtmittel, Gynäkologika, Hepatika, Hypnotika, Sedativa, Hypophysen-, Hypothalamushormone, regulatorische Peptide und Analoga sowie ihre Hemmstoffe, Immunmodulatoren, Kardiaka, Koronarmittel, Lipidsenker, Lokalanästhetika, Magen-Darm-Mittel, Migränemittel, Muskelrelaxanzien und - Reversoren, Narkosemittel, Neuropathiepräparate und andere neurotrope Mittel, Osteoporosemittel/Calcium-/Knochenstoffwechselregulatoren, Schilddrüsentherapeutika, Spasmolytika und Anticholinergika, Umstimmungsmittel, Urologika und Mittel zur Behandlung der Hyperkalämie und Hyperphosphatämie, Venentherapeutika, Wund- und Narbenbehandlungsmittel zur Anwendung gelangen.

11. Trägersysteme nach den Ansprüchen 1 bis 10 **gekennzeichnet dadurch, dass** die Konzentration von modifizierten Poly(dikarbonsäure-multiol estern) 0,1% bis 99,9999 % (m/m) beträgt.

## Claims

1. Injectable carrier systems based on modified aliphatic polyesters for use in humans or animals targeting the controlled release of a bioactive substance for therapeutic and/or diagnostic purposes consisting of polymers, the basic structure of which is **characterized in** a comb-like structure consisting of structural elements (A), (B) and (C);
where the polymer backbone is formed by the structural elements (A) and (B) consisting of aliphatic dicarboxylic acids (A) and polyols (B) containing three or more OH groups, wherein:
two hydroxyl groups of the polyol (B) with the dicarboxylic acids of the structural element (A) are esterified to form a linear polymer backbone, after formation of the polymer backbone consisting of the structural elements (A) and (B) and linked by ester bonds, one (for triols) or several (number of hydroxyl groups in the Polyol > 3) free hydroxyl groups are present that are fully or partially related to aliphatic fatty acids and/or
bioactive substances covalent or non-covalent (structural element (C)).

2. The carrier systems according to claim 1, **characterized in that** the aliphatic dicarboxylic acids of the structural element (A) of the following general structure 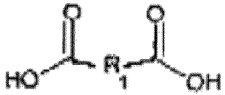 have a molecular moiety R₁ between the two carboxy groups, which has an alkyl structure and is saturated or unsaturated, branched or unbranched with R₁ = 0 (zero) and ≤ 40 (forty) carbon atoms, preferably 1 (one) to 14 (fourteen) carbon atoms, particularly preferably 4 (four) to 10 (ten) carbon atoms.

3. The carrier systems according to claim 1, **characterized in that** the polyol structural element (B) may consist of a mixture of triols and higher valent polyols or mixtures of the same.

4. The carrier systems according to claims 1 and 3, **characterized in that** glycerol, erythritol, xylitol, mannitol, sorbitol, lactose, sucrose, trehalose, glucose, maltose, isomalt and/or maltitol are used as the polyol.

5. The carrier systems according to claims 1, 3 and 4, **characterized in that** the polyol (B) is covalently linked to other substances by means of the structural element (C), so that a comb-like polymer structure is created, wherein the degree of linkage is between 0,1 and 100% of the free hydroxyl groups still present after the formation of the polymer backbone.

6. The carrier systems according to claims 1 to 5, **characterized in that** the structural element (C) is a carboxylic acid or one or more bioactive substance(s), which is (are) linked to the polyol structure (B) by an ester bond, the fatty acid being saturated or unsaturated and the degree of esterification being between 0 (zero) and 99.9% of the free hydroxyl groups.

7. The carrier systems according to claims 1 to 6, **characterized in that** the number of carbons of the carboxylic acid is between 2 (two) and 22 (twenty-two), in particular between 8 (eight) and 22 (twenty-two) carbon atoms, such as caprylic acid (octanoic acid), capric acid (decanoic acid), lauric acid (dodecanoic acid), myristic acid (tetradecanoic acid), Palmitic acid (hexadecanoic acid), stearic acid (octadecanoic acid), oleic acid ((9Z)-octadeca-9-enoic acid), linoleic acid ((9Z,12Z)-octadeca-9,12-dienoic acid), linolenic acid ((9Z,12Z,15Z)-octadeca-9,12,15-trienoic acid).

8. The carrier systems according to claims 1 to 7, in which the bioactive substance(s) is (are) incorporated (not covalently bound) or covalently bound, wherein, in case of incorporation (non-covalent binding), the bioactive substance(s) are present in dissolved, emulsified or suspended form, and in case of covalent binding, the bioactive substance(s) is (are) covalently bound to the polymer carrier material by a chemical reaction of one of the functional groups present in suitable functional groups of the bioactive substance(s), e.g., an acid group, with the free hydroxyl groups present in the structural element (B) is covalently bound to the polymeric carrier material by an ester bond.

9. The carrier systems according to claim 8, **characterized in that** the bioactive substances contained are hydrophilic, amphiphilic or hydrophobic in nature when having a low, medium or high molecular weight and are present in dissolved, emulsified or suspended form.

10. The carrier systems according to claims 8 and 9 **characterized in that** substances belonging to the class of antibiotics, antiinfectives, cytostatics or other antineoplastic agents and protectives, local anesthetics, analgesics, neuropharmaceuticals, hormones, immunomodulators, immunosuppressants, antifungals, anti-inflammatory agents, analgesics, anti-hemorrhagics, anti-fibrinolytics, vitamins, hemostatics, anti-inflammatory drugs, beta-receptor and calcium channel blockers, corticoids, withdrawal agents/agents for the treatment of addiction, fibrinolytics, anti-hypoxics, cardiovascular agents, anticoagulants, antiparasitic agents, anti-caries agents, antiperiodontics, ophthalmics, otologics, sex hormones and their inhibitors, antiparkinsonian agents and other agents for extrapyramidal disorders, psychopharmaceuticals, sera, immunoglobulins, vaccines, minerals, platelet aggregation inhibitors, tuberculosis agents, diuretics, gastrointestinal agents, anti-obesity agents, analeptics, antirheumatics, anti-allergics, anti-anemics, anti-arrhythmics, anti-dementia agents, antidiabetics, antidotes, antiemetics, antivertigo agents, antiepileptics, antihypertensives, anti-hypoglycemics, anti-hypotensive agents, antitussives, expectorants, anti-arteriosclerosis agents, inhibitors of the renin-angiotensin-aldosterone system, broncholytics, antiasthmatics and other respiratory tract agents, cholinergics, antiseptics, diagnostics and diagnostic preparation agents, diuretics, enzyme inhibitors, enzyme deficiency preparations and transport proteins, geriatrics, anti-gout agents, gynecologics, hepatics, hypnotics, sedatives, pituitary hormones, hypothalamic hormones, regulatory peptides and analogs and their inhibitors, immunomodulators, cardioactive agents, coronary agents, lipid lowering agents, local anesthetics, gastrointestinal agents, migraine agents, muscle relaxants and their reversors, anesthetics, neuropathic preparations and other neurotropic agents, osteoporosis agents/calcium/bone metabolic regulators, thyroid therapeutics, spasmolytics and anticholinergic agents, mood remedies, urologies and agents for the treatment of hyperkalemia and hyperphosphatemia, venous therapeutics, wound and scar treatments are used as bioactive substances.

11. The carrier systems according to claims 1 to 10 **characterized in that** the concentration of modified poly(dicarboxylic acid-multiol esters) is 0.1% to 99.9999% (m/m).

## Revendications

1. Systèmes supports injectables à base de polyesters aliphatiques modifiés, destinés à être utilisés chez l'homme ou l'animal, dans le but de libérer de manière contrôlée une substance bioactive à des fins thérapeutiques et/ou diagnostiques, constitués de polymères dont la structure de base est **caractérisée par** une structure en forme de peigne constituée des éléments structurels (A), (B) et (C),
dans lesquels le squelette polymère est formé par les éléments structurels (A) et (B) constitués d'acides dicarboxyliques aliphatiques (A) et de polyols (B) contenant trois groupes OH ou plus, dans lesquels
deux groupes hydroxyle du polyol (B) sont estérifiés avec les acides dicarboxyliques de l'élément structurel (A) pour former un squelette polymère linéaire, après formation du squelette polymère constitué des éléments structurels (A) et (B) et lié par des liaisons ester et lors de l'utilisation de triols et polyols de valeur supérieur dans l'élément structurel (B), il reste encore un (pour les triols) ou plusieurs groupes hydroxyle libres (nombre de groupes hydroxyle dans le polyol > 3) qui sont liés, en totalité ou en partie par covalence ou non, à des acides gras aliphatiques et/ou des substances bioactives (élément structurel (C)).

2. Systèmes supports selon la revendication 1, **caractérisés en ce que** les acides dicarboxyliques aliphatiques de l'élément structurel (A) de la structure générale suivante 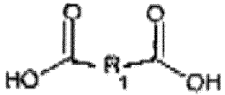 entre les deux groupes carboxyle présente une partie de molécule R₁ possédant une structure alkyle et étant saturée ou insaturée, ramifiée ou non ramifiée avec R₁ = 0 (zéro) et ≤ 40 (quarante) atomes de carbone, de préférence allant de 1 (un) à 14 (quatorze) atomes de carbone, de manière particulièrement préférée de 4 (quatre) à 10 (dix) atomes de carbone.

3. Systèmes supports selon la revendication 1, **caractérisés en ce que** l'élément structurel polyol (B) peut être constitué d'un mélange de triols et de polyols de valeur supérieure ou de mélanges de ceux-ci.

4. Systèmes supports selon les revendications 1 et 3, **caractérisés en ce que** les polyols utilisés sont le glycérol, l'érythritol, le xylitol, le mannitol, le sorbitol, le lactose, le sucrose (saccharose), le tréhalose, le glucose, le maltose, l'isomalt et/ou le maltitol.

5. Systèmes supports selon les revendications 1, 3 et 4, **caractérisés en ce que** le polyol (B) est lié par covalence à d'autres substances au moyen de l'élément structurel (C), de sorte qu'il se forme une structure polymère en forme de peigne, dans lequel le degré de liaison est compris entre 0,1 et 100 % des groupes hydroxyle libres encore présents après la formation du squelette polymère.

6. Systèmes supports selon les revendications 1 à 5, **caractérisés en ce que** l'élément structurel (C) est un acide carboxylique ou une ou plusieurs substances bioactives qui sont liées à la structure polyol (B) par une liaison ester, dans lesquels l'acide gras est saturé ou insaturé et le degré d'estérification est compris entre 0 (zéro) et 99,9 % des groupes hydroxyle libres.

7. Systèmes supports selon les revendications 1 à 6, **caractérisés en ce que** le nombre de carbones de l'acide carboxylique est compris entre 2 (deux) et 22 (vingt-deux), en particulier entre 8 (huit) et 22 (vingt-deux) atomes de carbone, tels que l'acide caprylique (acide octanoïque), l'acide caprique (acide décanoïque), l'acide laurique (acide dodécanoïque), l'acide myristique (acide tétradécanoïque), l'acide palmitique (acide hexadécanoïque), l'acide stéarique (acide octadécanoïque), l'acide oléique (acide (9Z)-octadéca-9-noïque), l'acide linoléique (acide (9Z,12Z)octadéca-9, 12-diénoïque), l'acide linolénique (acide (9Z, 12Z,15Z-octadéca-9, 12,15-triénoïque).

8. Systèmes supports selon les revendications 1 à 7, dans lesquels la ou les substances bioactives sont incorporées (non liées par covalence) ou sont liées par covalence, dans lesquels la ou les substances bioactives sont dissoutes, émulsifiées ou en suspension dans le cas d'une incorporation (liaison non covalente) et, en cas de liaison covalente, la ou les substances bioactives sont liées au matériau support polymère par une réaction chimique d'un groupe fonctionnel approprié de la ou des substances bioactives, par exemple d'un groupe acide, avec les groupes hydroxyle libres présents dans l'élément structurel (B) par une liaison ester.

9. Systèmes supports selon la revendication 8, **caractérisés en ce que** les substances bioactives de poids moléculaire faible, moyen ou élevé contenues sont de nature hydrophile, amphiphile ou hydrophobe et sont dissoutes, émulsionnées ou en suspension.

10. Systèmes supports selon les revendications 8 et 9, **caractérisés en ce que** les substances bioactives pouvant être utilisées sont les substances du groupe des antibiotiques, des anti-infectieux, des cytostatiques ou d'autres agents antinéoplasiques et des médicaments protecteurs, des anesthésiques locaux, des analgésiques, des neuromédicaments, des hormones, des immunomodulateurs, des immunosuppresseurs, des antifongiques, des anti-inflammatoires, des analgésiques, des anti-hémorragiques, des anti-fibrinolytiques, des vitamines, des hémostatiques, des anti-phlogistiques, des bêtabloquants et inhibiteurs calciques, des corticoïdes, des agents de sevrage/de traitement des addictions, des fibrinolytiques, des antihypoxémiants, des agents cardiovasculaires, des anticoagulants, des antiparasitaires, des agents anticaries, des agents de traitement de la parodontite, des médicaments ophtalmiques, des médicaments otologiques, des hormones sexuelles et leurs inhibiteurs, des agents utilisés dans le traitement de la maladie de Parkinson et autres agents utilisés dans le traitement des troubles extrapyramidaux, des psychotropes, des sérums, des immunoglobulines, des vaccins, des minéraux, des antiagrégants plaquettaires, des antituberculeux, des diurétiques, des agents gastro-intestinaux, des médicaments utilisés dans le traitement de l'obésité, des analeptiques, des antirhumatismaux, des antiallergiques, des antianémiques, des médicaments antiarythmiques, des médicaments antidémentiels, des médicaments contre le diabète, des antidotes, des antiémétiques, des antivertigineux, des antiépileptiques, des antihypertenseurs, des antihypoglycémiants, des antihypotenseurs, des antitussifs, des expectorants, des agents anti-artériosclérose, des inhibiteurs du système rénine-angiotensine-amidostérone, des broncholytiques, des médicaments contre l'asthme et autres agents pour les voies respiratoires, des médicaments cholinergiques, des antiseptiques, des médicaments diagnostiques et des agents de préparation de diagnostics, des diurétiques, des inhibiteurs enzymatiques, des préparations pour le déficit enzymatique et des protéines de transport, des médicaments de lutte contre le vieillissement, des médicaments pour la goutte, des médicaments gynécologiques, des médicaments pour le foie, des médicaments hypnotiques, des sédatifs, des hormones hypophysaires, des hormones hypothalamiques, des peptides régulateurs et analogues et leurs inhibiteurs, des immunomodulateurs, des médicaments pour le coeur, des agents coronariens, des hypolipémiants, des anesthésiques locaux, des agents gastro-intestinaux, des agents utilisés dans le traitement de la migraine, des myorelaxants et myoinverseurs, des anesthésiques, des préparations contre les neuropathies et autres agents neurotropes, des agents utilisés dans le traitement de l'ostéoporose/des régulateurs du métabolisme calcique/osseux, des agents thérapeutiques pour les maladies de la thyroïde, des anticonvulsants et des médicaments anticholinergiques, des régulateurs d'humeur, des médicaments urologiques et des agents pour le traitement de l'hyperkaliémie et de l'hyperphosphatémie, des agents thérapeutiques veineux et des produits pour le traitement des plaies et des cicatrices.

11. Systèmes supports selon les revendications 1 à 10, **caractérisés en ce que** la concentration en poly (esters multiols d'acide dicarboxylique) modifiés est de 0,1 % à 99,9999 % (m/m).
